# EUROPEAN PATENT SPECIFICATION

(11) **EP 1 317 944 B1**
(45) Date of publication and mention of the grant of the patent: **25.10.2006**
(21) Application number: 01830747.0
(22) Date of filing: 06.12.2001
(51) Int. Cl.: A61N 5/10, G21K 5/04, H05H 7/22

(54) **Apparatus for the intraoperative radiation therapy for the linear acceleration of electrons**
Vorrichtung zur Elektronenlinearbeschleunigung während einer intraoperativen Strahlungstherapie
Dispositif pour l'accélération linéaire d'électrons dans la radiothérapie peropératoire

(43) Date of publication of application: 11.06.2003
(73) Proprietor: HITESYS S.p.A., 04011 Aprilia (Latina) (IT)
(72) Inventor: Venier, Luigi, 04011 Aprilia (LT) (IT); Santoni, Felice, 04011 Aprilia (LT) (IT); Sagripanti, Alberto, 04011 Aprilia (LT) (IT)
(74) Representative: Modiano, Micaela Nadia

(56) References cited:
- EP-A- 0 702 982
- WO-A-97/13552
- US-A- 2 910 601
- US-A- 5 321 271
- US-A- 5 810 707

## Description

The present invention relates to the technological innovations of the "apparatus for the linear acceleration of electrons, particularly for intraoperative radiation therapy" that the Hitesys S.p.a brought in ( whose patent N° 01281184, awarded in Italy in date 02.17.1998, and US-A- 5635721, awarded in U.S.A. on 06.03.1997, and European Patent Office application EP-A- 0 702 982.
The present invention and patents recalled and appointed are applied to the intraoperative radiation therapy is a therapeutic method used in treating deep neoplasm and consists in delivering a single intense dose of radiation onto a tumoral mass, preventing the dose from affecting the surrounding healthy tissues.Usig the present invention, by delivering the dose of radiation directly onto the tumour or onto the macroscopic or microscopic tumoral residue, it is possible to spare the peritumoral healthy tissues that are instead affected by radiation in conventional radiation therapy with external beams.

Its flied of utilisation ranges from surgically inoperable tumours, to tumoral residues after partial surgical exeresis, to a tumoral bed after full surgical removal. For a wide range of tumours, particularly those affecting the abdomen, the pelvis, and the chest,an generally affecting... use of electron-beam therapy offers high versatility in treating tumoral residues after surgical removal as well as tumoral masses deemed inoperable.
The present invention very much improves the main working features described in the patents recalled and appointed, whose contribution for developing the intraoperative radiation therapy is to be found into researches that the applicant Society carried on long, reported later on in the present description.
the patents recalled and appointed intended to eliminate many disadvantages and/or risks for the use of the linear acceleration electrons, in the specific field of the intraoperative radiation therapy, which:
-- the need transfer the patient to a location other tan the operating room causes problems linked to the risks of transferring the patient under anaesthesia and to the time that elapses between surgical exeresis and subsequent radiation therapy;
-- the need to strictly schedule each operation according to the availability of access to the radiation-therapy site, and this increases the working time requirements ad reduces the number of patient who can utilise this radiation therapy;
-- the high manufacturing cost and the remarkable encumbrance of the apparatus; the impediment to placed the radiating head in space and to be moved in a flexible manner, so that the electron beams treats the entire tumoral mass involved, despite the irregular shape that said mass may have;
-- the elimination of the intense irradiation produced, which cannot be confined with simple movable panels, and therefore in the consequent need for heavily shielded work sites.

For the elimination of the disadvantages and/or risks, the patent ahead recalled shows and shape the realisation of the apparatus for the linear acceleration of electrons, particularly for intraoperative radiation therapy, that:
- can by used directly in the operating room without special radiation-protective measures; that allows the flexible and precise movement in space of the electron beam to treat tumoral masses having variable and different shapes;
- allows to provide the electron acceleration section separately from the radio-frequency generation ad control section;
- allows to achieve a very low X-ray level that can by easily shielded; that allows to spatially vary the dose of radiation that is incident to a given tumoral area; having a modest size and weight;
- avoids the need for the external focusing an centring devices for the emitted electron beam.
- avoids to eliminate cathod from the accelerating structure.

All the innovations of the patents, ahead recalled and appointed, and that in the specific flied of the itraoperative radiation therapy define the current state of the technique, find in the present invention all indication to improveof the working features and/or of the working requirements in reply to present needs of the itraoperative radiation therapy.
Particulary the present invention keeps functions and/or working capability of the patents recalled and appointed , introducing anyway some improvements as results of detailed researches about medical field.
In short the improvements relate to:
- the presence of a radiation shield under the treatment couch, whose centring system depends on an ultrasound trasductors sistem or such like;
- the new configuration of the articulated structure, aiming to make easy the RF transmission to the accelerating structure, without introducing mechanical oppositions to the pitching motions of the radiating head and rolling motions of the whole group;
- the new configuration of the radiating head performing the spherical motion of the radiating head with its centre on the axis of the acceleration structure and having preferabily a barycentric position to minimize the inertial moment;
- the placing of modulator in any part of the movable system and anyhow on the edge of the movable system, because the new configuration forsees the passage of the wave guide , which transmits the RF to the acceleration structure, within the structure of the forked bearing;
   The innovations introduced with the present invention are indicated and described later on toghether with the keeped features, to give prominence to all the working features of the apparatus for intraoperative radiation therapy.
   A principal aim of the present invention is therefore to provide an apparatus for the linear acceleration of electrons, particularly for intraoperative radiation therapy, which can be used directly in the operating room without special radiation-protective measures, that, on the grounds of the techniques fits in the patent ahead called, introduce other technical elements more advanced for the realisation of the apparatus more satisfactory for the running, furniture, autonomy and control of the process radio-therapeutic, whatever proposals may be.
   Within the scope of this aim, an object of the present invention is to provide an apparatus for the linear acceleration of electrons that allows too of to enliven through radio control the whole articulated structure, in order to manage jointly the radiating head, but also shift's comfort outside and in operative room, for allows better the flexible and precise movement in space of the electron beam to tat tumoral masses having variable and different shapes.
   Another object of the present invention is to provide an apparatus for the linear acceleration of electrons feed from uninterruptible power supply, that give called apparatus independent, during therapeutic process, from the power supply network for to avoid interference and/or troubles of the network to the functions of the same apparatus.
   A further object of the present invention is to provide an apparatus for the linear acceleration of electrons having the frequency modulator in the horizontal arm of the support of the articulated structure holding the radiating head, or in any other part of the movable system. Such placing allow a better flexibility and a certain balance of the apparatus.
   Another further object of the present invention is to provide an apparatus for the linear acceleration of electrons having a automatic frequency control, that objet is to keep the oscillation frequency of the magnetron always coincidental with that of resonance of the linear accelerator of electrons structure.
- Another further object of the present invention is to provide an apparatus for the linear acceleration of electrons, in addition to those described in the patents recalled ed appointed, having a structure able to give scannign motion to the radiating head and to set it in a spherical motion with the centre on the axis of the acceleration structure to minimize the inertial moment;
   Not last object of the present invention is to provide a cheap apparatus for the linear acceleration of electrons of high reliability and easy production.
   This aim, these objects, and others which will become apparent hereinafter are achieved by an apparatus for the linear acceleration of electrons, particularly for intraoperative radiation therapy, as defined in claim 1.

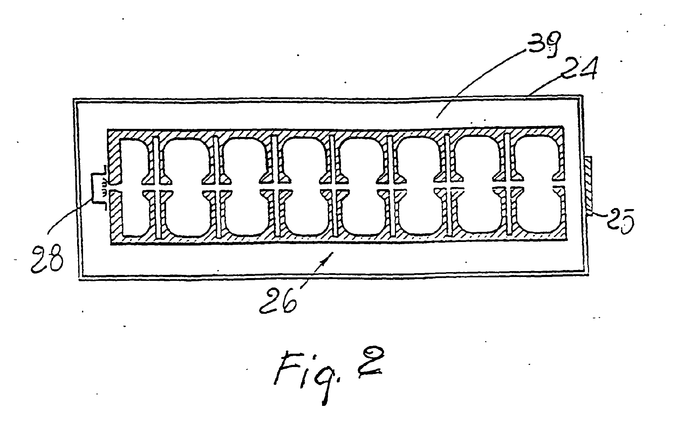

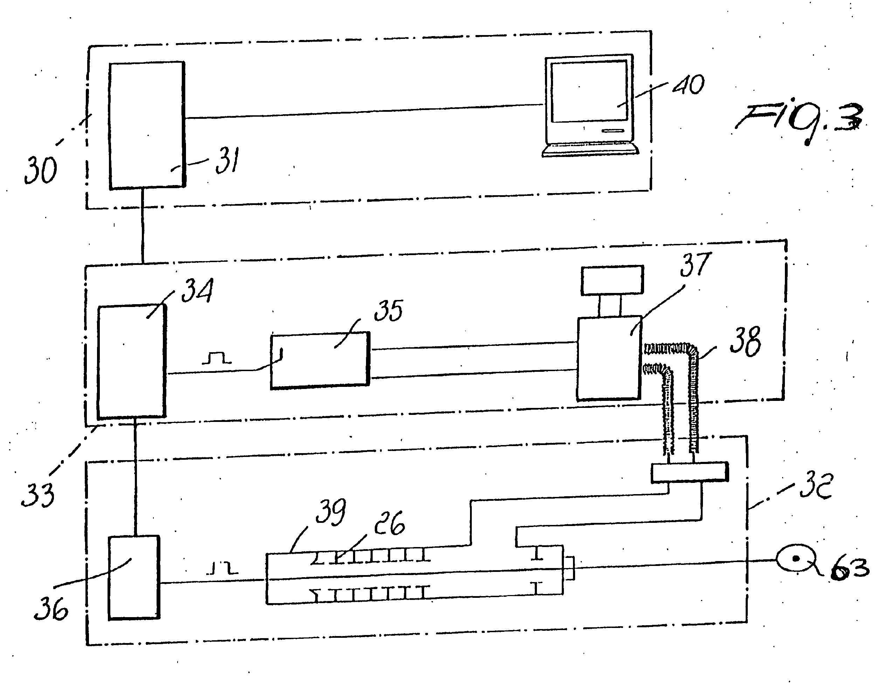

Further characteristics and advantages of the invention will become apparent from a preferred but not exclusive embodiment of the apparatus to the invention, illustrated oily by way of non-limitative example in the accompanying drawing, wherein:

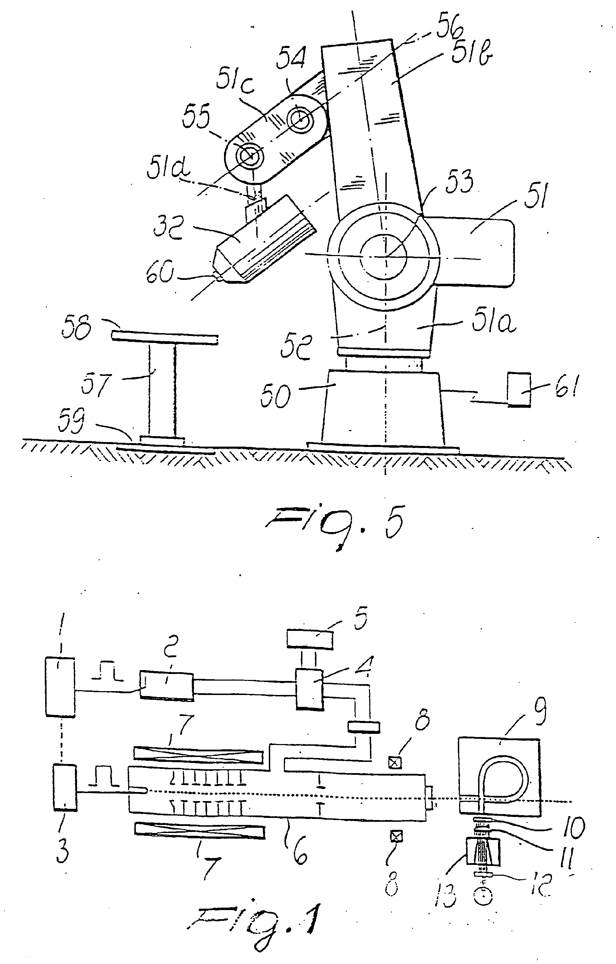

figure 1 is a view of a known type of apparatus for the linear acceleration of electrons;
figure 2 is a view of the configuration of the acceleration cavities for a known type of acceleration apparatus;
figure 3 is a block diagram of a known type of an apparatus for the linear acceleration (patents recalled and appointed);
figure 4 is a block diagram of automatic frequency control system according to the known type of acceleration apparatus (patents recalled and appointed);
figure 5 is a lateral elevation view of a robot and of radiating head, said robot and said head being a part of acceleration apparatus of a known type;
figure 6 is a lateral elevation view of a robot and of radiating head, said robot and said head being a part of the present acceleration apparatus;
figure 7 shows the radiating head gruop according to the present invention;
figure 8 is a flowchart of the steps for the characterisation of area of the body of a patient to be treated with the apparatus of a known type(patents recalled and appointed);
figure 9 is a flowchart of the operating steps for scanning irradiation by using the apparatus of a known type (patents recalled and appointed). With reference to figure 1, the known acceleration apparatus comprises a radio-frequency modulator 1, a magnetron 2, cathode modulation means 3, circulation means 4, a load cooling water 5, a acceleration structure 6, a focusing magnet 7, a centring magnet 8, a deflector magnet 9, a beam diffuser 10, a beam equaliser 11, a beam applicator 12, and a beam collimator 13.
   With references instead to figures 2 and 3, is illustrated in detail the known and patented acceleration structure , which is constituted by a plurality of acceleration cavities 26 ( also known as resonant cavities), arranged in series one next to the other, by a cathode 28 connected to the first acceleration cavity and by control and processing means 30. The acceleration cavities 26 are enclosed by an external vacuum-tight jacket 24.

The acceleration cavities 26 of the acceleration structure of a known type 39 are designed so as to produce radio-frequency self-focusing along the X-axis of said cavities.

Self-focusing has bee obtained by using different length values for the first, second, third, fourth and fifth acceleration cavities, so that the lengths increase from the first cavity to fifth one and are constant for the subsequent cavities. Particularly, it has been found that the optimum lengths for the first, second, third, fourth an fifth cavities are 25 mm, 40 mm, 45 mm, 48 mm and 50 mm, respectively. The energy of the electrons and their capture have also been treated for the first cavity, in which the electrons are not yet relativistic ad the values of b and r (Lorenz parameters) vary appreciably.

A cathode 28 is placed in front of the first acceleration cavity and is supplied by the cathode modulation means 36; a thin titanium lamina is arranged outside the last acceleration cavity for vacuum tightness.

The control and processing means 30 comprise: power supply means 31; processing means 40, which advantageously comprise computer; the cooling system of the apparatus; the means for distributing motive power; the safety devices (not shown).

The control and processing means 30 are connected to modulation means 33 that comprise radio-frequency modulation means 34 connected to magnetron 35 and to cathode modulation means 36. The magnetron 35 is protected from accidental load reflections by a ferrite isolating system 37. Waveguide means 38, conveniently constituted by a flexible waveguide, connect the radio-frequency modulation means 33 to irradiation means, constituted by a radiation head 32 that comprises an acceleration structure 39.

In figure 3, the reference numeral 63 designates a particular area of the body of the patient that must be treated by irradiation by means of the apparatus of a known type and according to the invention.

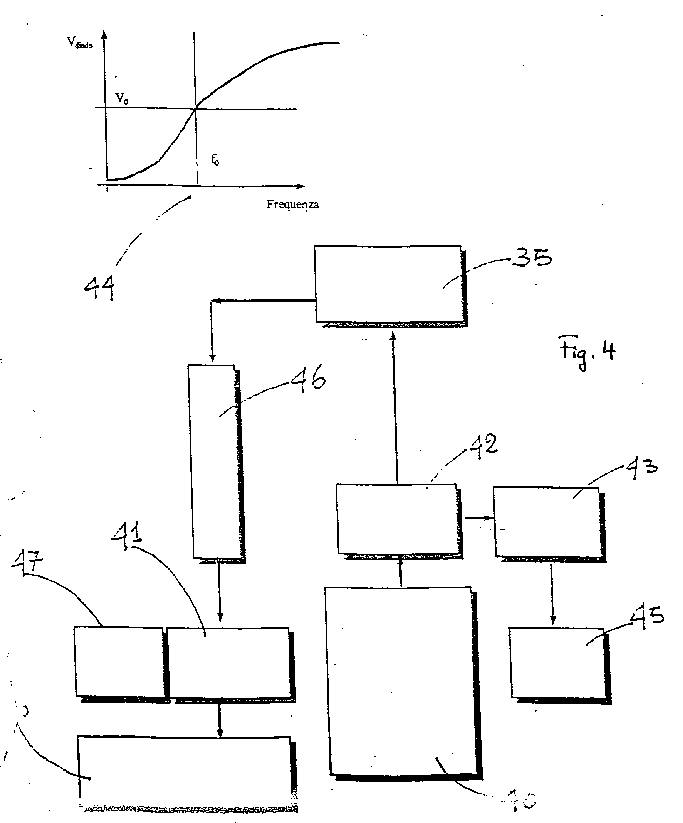

With reference instead to figure 4, the acceleration apparatus according to the invention comprises also the radio-frequency self- control, of which object to keep the frequency of oscillation of the magnetron 35 coinciding with that of the resonance of the acceleration structure 26.
In according of the known type of the apparatus, as a matter of fact, the magnetron 35 and the acceleration structure 26 undergo to frequency individual deviations of the work point ( within limits), what for example the temperature, what influences the resonance frequency.

The present invention eliminate such disadvantage by means of a self-control circuit of the frequency 40 that provides to keep in step the oscillator and load.

A capacity coupling 41, placed in the knot of the wave guide 38, draws a share of the radio-frequency. In fact, the feature of the knot point is that to present a behaviour of the voltage highly depending from the type of coupling oscillator-load; such dependence is showed in the graph 44. In the abscissas axis are carried the emission frequencies of the magnetron 35; in the middle of the axis is gave the resonance frequency of the accelerator f₀. The axis of ordinate represents the voltage took from the diode joined with the capacity coupling 41: peak value of the voltage present in the wave-guide 38, in the knot point where the coupling is inserted.

Varing the frequency of the magnetron, the voltage took from the diode to vary around a central value V₀; confronting afterwards the voltage of the diode with an voltage equal to V₀, it is possible to dispose of a signal right for to drive the motor that the syntony axis, 42; the rotation towards is that what stretch to carry V toward V₀ and so therefore the frequency f toward f₀.

Afterwards the peculiar characteristic of the frequency self-control circuit analysed is that to make use only one signal drew from the wave guide, unlike of the known system and of all the other sisters that use 2 signals.

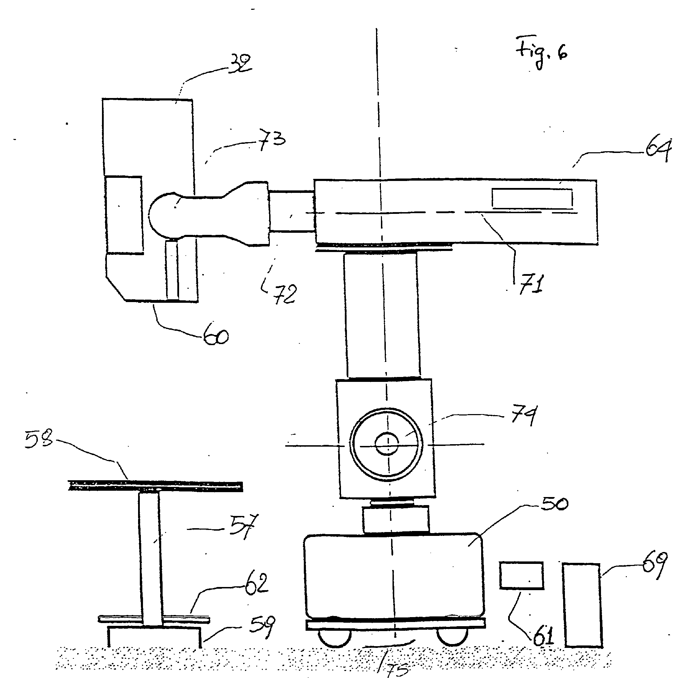

Figure 5 is a lateral elevation wives of the apparatus of a known type in which the reference numeral 50 designates a supporting structure fixed to the floor or resisting thereon.

An articulated structure 51 is located on the supporting structure 50 and is meant to support and move the radiating head 32; a diaphragm 60 is applied to said radiating head in the position where the electronic beam exits.

In detail, the articulated structure 51, commonly termed tube support, is constituted by a vertically arranged robot comprising four articulated and mutually interconnected segments designated by the reference numerals 51a, 51b, 51c and 51d, which allow to arrange the radiating head 32 in any spatial position.

The articulated segments 51a, 51b, 51c and 51d are mutually pivoted so as to give the radiating head 32 six degrees of freedom in space.

In particular, the articulated segment 51a is routable about the rotation axis 52 of the supporting structure 50 both clockwise and counterclockwise; the articulated segment 51b is routable in both directions about the hinge axis 53; the segment 51c is routable in both directions about the hinge axis 54 as well as about the axis 56; and the segment 51d is routable in both directions about the hinge axis 55.

The reference numeral 57 designates a supporting post for operating table 58. Said post rests on a footing 59 that is moderately shielded, with respect to the floor, only if access to the rooms beneath the operating room is not prohibited during use of the apparatus of a known type. Said shielding is not necessary otherwise.

Control means, advantageously comprising a movable button panel 61, control the apparatus of a the known tie.

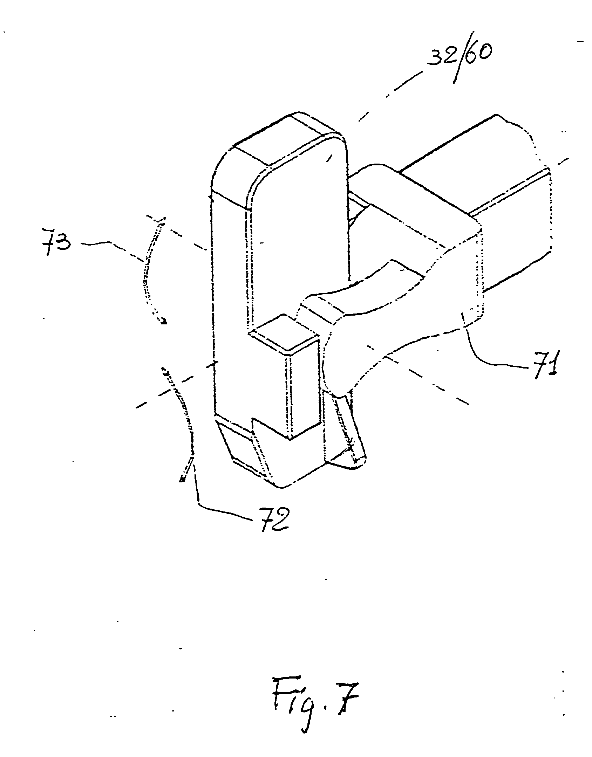

Figure 6 is a lateral elevation view of the apparatus according to the invention, in which the reference numeral 50n (new) designates a supporting structure with carriage self-propelled and radio controlled as all other components of the supporting structure of the radiating head 32, that keeps six degrees of freedom in space, as in figure 5.

Considering that the fundamental working principles of the known type of the apparatus, the reference numerals previously described correspond just to the same things, while are carried the reference numerals regarding the functions or the inserted parts of the present invention.

As regards, the figure 6 show the better placing of the modulator (34,36), housed in a metal container entirely closed 64 placed in the horizontal arm of the articulated structure or in any other part of the movable system, for the generation of the radio-frequency to send to the cavities 26 of the articulated structure of the robot 39.

Furthermore, with the reference numeral 62 is showed the integrated radiatin shield of the electrons beam whose aim is to centre said electrons beam during the opearation.
And some more, although not showed in figure 6, for the improvement that to object the present invention, has been introduced a thermostatic circuit with a exchanging heat air-fluid instead of water-fluid. Obvious, the fluid used for the cooling is alone that of the shunt circuit of the apparatus and not there are necessities of the external intake.

After all that, with reference to figure 6, has been shown the numbers of the part-functions inserted and/or altered from the present invention, preserving the numeration of the part-functions not altered, as in figure 5. Figure 7 shows the new configuration of the radiating head 60 as a system performing said spherical motion using a spherical forked bearing 71 and by combining two rotation motions with incident orthogonal axis: rotation of the forked bearing with respect to the frame 72 ( rolling motion of the forked bearing and of the head); rotation of the head with respect to the forked bearing 73 ( pitching motion of the head);

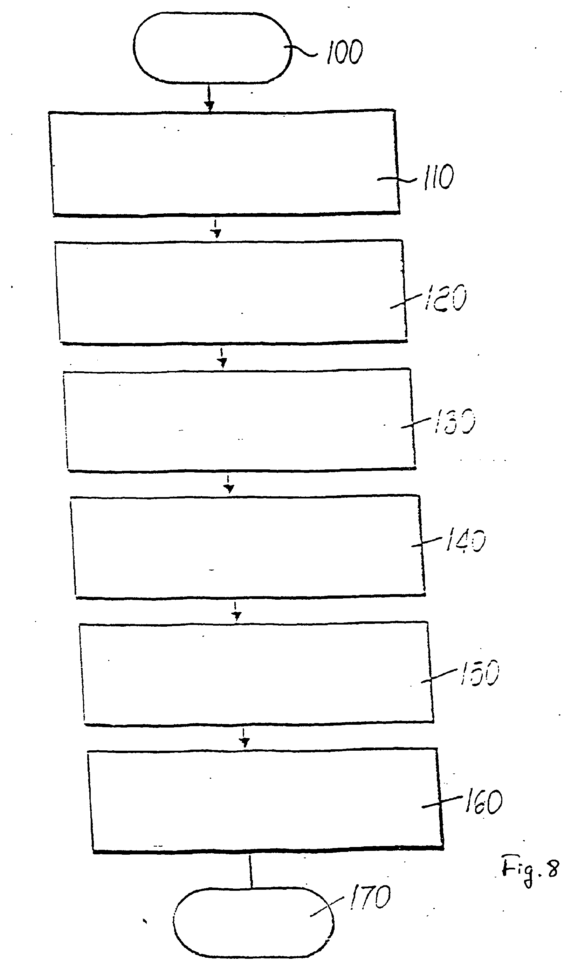

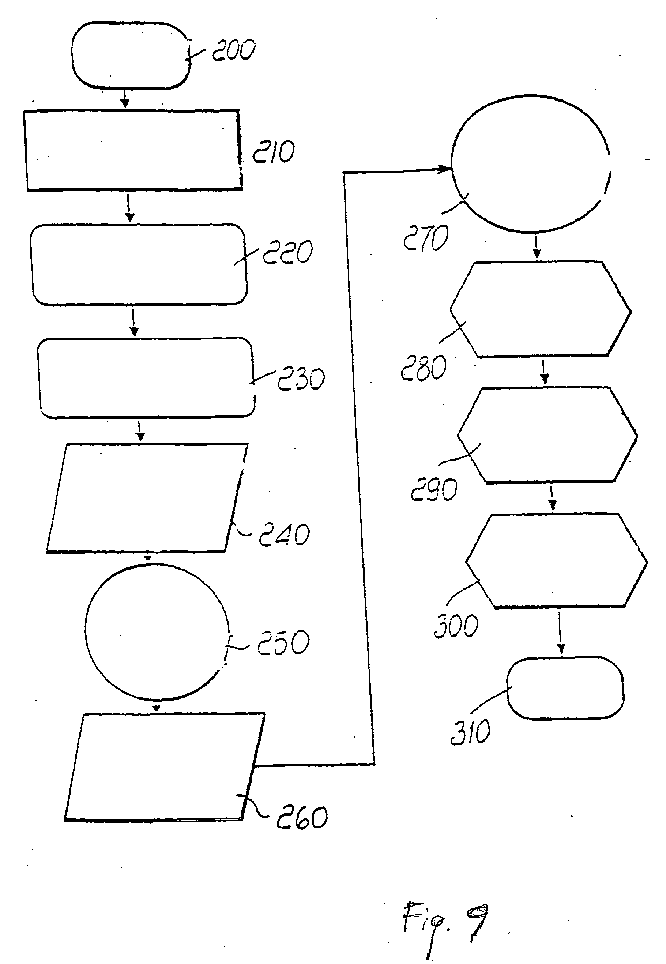

Figures 8 and 9 are flowcharts of the operating steps of the apparatus according to the invention, which are handled by processing means 40.

With reference to the above figures, the operation of the apparatus for the linear acceleration of electrons according to the invention is as follows.
The carriage robot constituted by the articulated segments 71,72,73,74 e 75 allows to orientates the radiating head 32 so as to direct the electron beam exactly onto the region where the therapy is to be performed. The possibility of orientating the radiating head 32 allows to avoid expanding the beam and therefore allows precise treatment of the region to be irradiated, bringing the radiating head 32 very close to the region to be irradiated. This, in comparison with the minimum possible distance that can be obtained with known linear accelerators, which varies from 80 to 100 cm, allows the apparatus according to the invention to have a much higher efficiency of the aid patent, since there is no beam scattering and a much lower power level is thus required (from 4 to 12 MeV).

Furthermore, the carriage supporting structure self-propelled and radio controlled 50n, according to the invention, consent the approach of the apparatus to the supporting post for operating table 58. Shifts what are radio-controlled and monitored respect to tumoral area of the patient that must be took by irradiation to the apparatus of the known type and according to the invention 63.

Continuous measurement of the position of the various articulated segments that constitute the robot occurs by means of a sensor system (not shown) with which the robot is equipped.
The processing means 40 allow to predefine the desired movements of the articulated segments 72,73,74 and 75 that constitute the robot, and therefore of radiating head 32, so that said radiating head closely follows the contour of the region to be irradiated; it is furthermore possible to set the desired doses of radiation for each point of the region to be treated.

The modulation means generate and control the radio-frequency and feed to the cathode 28. The generated and self-controlled radio-frequency 40 is sent to the acceleration cavities by means of the flexible wave-guide 38.

The electrons that move along the axis of the acceleration cavities 26 are gradually accelerated by the radio-frequency flied inside each cavity 26 until they reach the desired final energy. The electrons exit from the acceleration structure 32 through the thin titanium lamina 25, whose thickness whereof allows the electrons to pass there through without losing an appreciable part of the energy they possess.

The radio-frequency electric field used to accelerate the electrons is produced by the magnetron 35, controlled 40, which feeds the acceleration structure 32 by means of the wave-guide 38.

The modulator of the cathode 36 feeds the cathode and synchronises its operation so that the train of radio-frequency pulses that feeds the acceleration structure 32 is matched by emission of electrons on the part of the cathode.

The beam of electrons is focused an accelerated simultaneously in the first cavities with a set combination of the cathode injection energy and of the length of the first, second, third, fourth an fifth cavities, and can pass through the centre of the subsequent cavities of the acceleration structure after the peak of the radio-frequency, so as to undergo additional focusing.

The beam of electrons is pulsed, and each pulse lasts 4 microseconds. The frequency of the pulses can be fixed or variable allowing to use the apparatus, according to the present invention,directly inside operating room without special radiation-protective measures.

Use of the processing means 40 allow to use the apparatus according to the invention for mechanical scanning irradiation comprise four different operating states of the acceleration apparatus.

These four states are:
-- instruction step;
-- learning step;
-- verification step;
-- therapy step.
Figure 8 is a block diagram of the sequence of steps for defining an area to be irradiated, which is called "source plane", performed by the processing means 40; in said figure, after the initial step 100, there is a step 110 for selecting the plane on which the area to be irradiated lies; the step 110 is followed by the step 120 for selecting the inclination angle of the radiating head 32 with respect to the defined source plane; this is then followed by the step 130 for entering the vertices of the figure to be irradiated, the step 140 for tracing the perimeter of the figure for confirmation, the step 160 for calculating the data for irradiation, the irradiation step 150, and finally the end step 170.

With reference now to figure 9, when the patient is ready for electron therapy ( step 200), the apparatus according to the invention is placed (step 210), by opening the articulated structure 51, so that its beam direction indicator is trained on the centre of the area to be treated. This movement is controlled by means the movable bottom panel 61.

At this point, the apparatus is placed in the learning state: the operator starts to move the beam over a path that coincides with the edge of the region to be treated; this path is developed over more or less spaced points, depending on the complexity of the profile, and the processing means 40 interpolate an connect the various points with straight segments or circular arcs.

Once learning has ended, the apparatus is placed in the verification step, during which the operator enters ( steps 240 and 260), the required dose and the corresponding energy for each field and the articulated structure 51 constantly moves ( steps 250 and 270) along the corresponding paths with its light beam.

Once the verification step has ended, the steps for the irradiation of the various fields (steps 280, 290 and 300) occur. These irradiation steps can be followed on the monitor of the processing means 40, which displays data related in real time to the path in progress, the percentage of treatment performed, the dose given, and the remaining dose.

This is followed by the end step 310.

In the case of conventional irradiation, the operator, after moving the radiating head 32 close to the patient, and after setting the desired dose by virtue of the processing means 40, moves the radiating head 32 towards the collimator cone that is used to protect the parts of the patient's body that are non to be irradiated from the electrons beam, and begins the irradiation.
The separation between the radiating head 32 and the modulation means 33, and their connection by means of the flexible wave-guide 38, allow to provide an articulated mechanical arm 51 capable of positioning and moving in space the acceleration structure 39 and the radiating head 32 with extreme precision and in very flexible manner.
Control by means of an appropriately programmed computer 40 allows to set up the movement of the articulated structure 51 so that the beam of electrons treats the entire tumoral mass, no matter how irregular it might be.

The beam self-focusing characteristic, combined with the shape of the acceleration structure 32, make it unnecessary to have auxiliary external devices for regulating the beam (magnets, etceteras), thus allowing a very low X-ray level. This reduction in radiation allow using the apparatus in operating rooms without particular shielding.

The use vacuum-tight welding to assemble the various acceleration cavities 26 that constitute the acceleration structure 32 allows eliminating the external vacuum-tight jacket, thus reducing weight and size.

The device thus conceived is susceptible of numerous modifications and variations, all of which are within the scope of the inventive concept. Thus, for example, since in the apparatus according to the invention the cross-section of the beam remains very small, the current of the beam must in turn be reduced considerably, on penalty of risking the delivery of an excessive local dose and therefore necrotizing the irradiated tissue. The need to reduce the intensity of the current can allow eliminating the cathode 28 from the acceleration structure 39 by virtue of known phenomenon of cold extraction of electrons from a metallic material. This phenomenon consist of the fact that an intense electric field applied to a metallic material is able to extract a certain number of electrons from the outermost atomic orbits. The number of electrons that can be extracted, however, is not sufficient for the beam currents required by known accelerators: with certain exception, as the patent ahead mentioned.

Finally, all the details may be replaced with other technically equivalent elements. In practice, the materials employed, so long as they are compatible with the specific use, as well as the dimensions, may by any according to the requirements and state of the art.
Where technical features mentioned in any claim are followed by reference signs, those reference signs have been included for the only purpose of increasing the intelligibility of the claims and accordingly such reference signs do not have any limiting effect on the interpretation of each element identified by way of example by such reference signs.

## Claims

1. An apparatus for the linear acceleration of electrons, particularly for intraoperative radiation therapy, comprising
- irradiation means having an acceleration structure constituted by a plurality of cavities (26) and a radiating head (32);
- an articulated structure (51) for moving said irradiation means;
- modulation means (33) for generating, self-controlling and transmitting a radio-frequency to said cavities of said acceleration structure (51);
- processing and control means (30) adapted to control said apparatus, said modulation means being separate from said irradiation means, the connection occurring through a flexible wave-guide (38) adapted to carry the radio-frequency to said acceleration structure (51);
- a forked bearing (71) supporting the radiating head (32) connected to the articulated structure (51) by means of a rotation wheel for performing a rolling motion of the forked bearing (71) and of the radiating head (32), within which a rotating joint and a section of the flexible wave guide (38) are located; the radiating head (32) being connected to the forked bearing (71) through a pivot having its axis orthogonal to the rolling axis for performing pitching motions;
- a radiation shield under a treatment couch (58) comprising a centering system.

2. An apparatus according to claim 1, wherein said articulated structure (51) comprises a robot for the movement of said irradiation means, said robot being arranged on a mobile supporting structure self-propelling above the floor of the operating room.

3. An apparatus according to claim 2, wherein said robot comprises four articulated segments that are movable with respect to each other and allow said irradiation means to move with six degrees of freedom.

4. An apparatus according to claim 1, wherein said plurality of cavities of said acceleration structure are interconnected by means of vacuum-tight braze welds, the cavities from the first to the fifth one having increasing lengths, the subsequent cavities being identical in length, said plurality of cavities producing a self-focusing of the electron beam.

5. An apparatus according to claim 4, wherein said acceleration structure comprises a cathode located in front of said first cavity and titanium lamina located outside the last cavity.

6. An apparatus according to claim 1, wherein said modulation means comprise a magnetron, a radio-frequency modulator, and a cathode modulator adapted to enable said cathode.

7. An apparatus according to claim 4, wherein said acceleration structure comprises a titanium lamina located outside the last cavity.

8. An apparatus according to claim 1, wherein said modulation means comprise a magnetron and a modulator of radio-frequency placed in an arm of the articulated structure.

9. An apparatus according to claim 6, wherein said modulation means comprise an automatic control of the frequency to keep the oscillation frequency of the magnetron coinciding with that of the resonance of the acceleration structure.

## Patentansprüche

1. Eine Vorrichtung zur Elektronenlinearbeschleunigung, insbesondere für die intraoperative Bestrahlungstherapie, das folgendes umfaßt:
- Bestrahlungmittel, das eine Beschleunigungsstruktur hat, das aus einer Mehrzahl von Hohlräumen (26) und einem Strahlungskopf (32) gebildet ist;
- eine Gelenkstruktur (51) zum Bewegen des Bestrahlungsmittels;
- Modulationsmittel (33) zum Erzeugen, Selbstüberwachen und Übertragen einer Hochfrequenz zu den Hohlräumen von der Beschleunigungsstruktur (51)
- Verarbeitungs- und Kontrollmittel (30), die angepaßt sind die Vorrichtung zu steuern, wobei das Modulationsmittel von dem Bestrahlungsmittel getrennt ist, wobei die Verbindung durch einen flexiblen Wellenleiter (38) stattfindet, der angepaßt ist, die Hochfrequenz zu der Beschleunigungsstruktur (51) zu leiten;
- eine gegabelte Stütze (71), die den Strahlungskopf (32) trägt, die mit der Gelenkstruktur (51) mittels einer Drehwelle verbunden ist, um eine Drehbewegung der gegabelten Stütze (71) und des Strahlungskopfes (32) auszuführen, worin sich ein drehbares Gelenk und ein Teil des flexiblen Wellenleiters (38) befinden; wobei der Strahlungskopf (32) mit der gegabelten Stütze (71) durch eine Drehachse, die ihre Achse orthogonal zur Rollachse hat, mit der gegabelten Stütze (71) verbunden ist, zur Ausführung von Schwingbewegungen.
- einen Strahlungsschirm unter einer Behandlungscouch, umfassend ein Zentrierssystem.

2. Eine Vorrichtung gemäss Patentanspruch 1, worin die Gelenkstruktur (51) einen Roboter zur Bewegung des Bestrahlungsmittel umfasst, wobei der Roboter auf einer mobilen Stützstruktur angeordnet ist, die sich selbstangetrieben über dem Boden des Operationsraumes bewegt.

3. Eine Vorrichtung gemäss Patentanspruch 2, worin der Roboter vier Gelenksegmente umfasst, die in Bezug aufeinander beweglich sind und dem Bestrahlungsmittel erlauben, sich mit 6 Freiheitsgraden zu bewegen.

4. Eine Vorrichtung gemäss Patentanspruch 1, worin die Mehrzahl von Hohlräumen der Beschleunigungsstruktur untereinander mittels vakuumdichter Lötnähte verbunden sind, wobei die Hohlräume von dem ersten bis zum fünften zunehmende Längen haben, die nachfolgenden Hohlräume identisch in der Länge sind, wobei die Mehrzahl von Hohlräumen eine Selbstfokussierung des Elektronenstrahls erzeugt.

5. Eine Vorrichtung gemäss Patentanspruch 4, worin die Beschleunigungsstruktur eine Kathode umfaßt, die vor dem ersten Hohlraum angeordnet ist, und eine Titanschicht, die ausserhalb des letzten Hohlraums angeordnet ist.

6. Eine Vorrichtung gemäss Patentanspruch 1, worin das Modulationsmittel ein Magnetron, einen Hochfrequenzmodulator und einen Kathodenmodulator umfasst, das angepaßt ist, die Kathode zu betreiben.

7. Eine Vorrichtung gemäss Patentanspruch 4, worin die Beschleunigungsstruktur eine Titanschicht umfasst, die ausserhalb des letzten Hohlraums angeordnet ist.

8. Eine Vorrichtung gemäss Patentanspruch 1, worin das Modulationsmittel ein Magnetron und einen Hochfrequenzmodulator umfasst, das in einem Arm der Gelenkstruktur untergebracht ist.

9. Eine Vorrichtung gemäss Patentanspruch 6, worin das Modulationsmittel eine automatische Frequenzkontrolle umfasst, um die Oszillationsfrequenz des Magnetrons in Übereinstimmung mit der von der Resonanz der Beschleunigungsstruktur zu halten.

## Revendications

1. Dispositif pour l'accélération linéaire d'électrons, en particulier dans la radiothérapie intra-opératoire, comprenant
- des moyens d'irradiation possédant une structure d'accélération constituée d'une pluralité de cavités (26) et d'une tête de radiation (32);
- une structure articulée (51) pour déplacer lesdits moyens d'irradiation;
- des moyens de modulation (33) pour générer, auto-contrôler et transmettre une radiofréquence auxdites cavités de ladite structure d'accélération (51);
- des moyens de contrôle et de traitement (30) adaptés pour contrôler ledit dispositif, lesdits moyens de modulation étant séparés desdits moyens d' irradiation, la connexion survenant à travers un guide d'ondes flexible (38) adapté pour amener la radiofréquence à ladite structure d'accélération (51);
- un palier fourché (71) supportant la tête de radiation (32) relié à la structure articulée (51) au moyen d'une roue de rotation pour l'accomplissement d'un mouvement de roulement dudit palier fourché (71) et de ladite tête de radiation (32), dans laquelle un joint de rotation et une section dudit guide d'ondes flexible (38) sont situés; la tête de radiation (32) étant connectée au palier fourché (71) à travers un pivot ayant son axe orthogonal à l'axe de roulement pour l'accomplissement de mouvements d'inclinaison;
- un bouclier de radiation sous une table de traitement (58) comprenant un système de centrage.

2. Dispositif selon la revendication 1, dans lequel ladite structure articulé (51) comprend un robot pour le mouvement desdits moyens d'irradiation, ledit robot étant agencé sur une structure de support mobile auto-motrice sur le plancher de la salle opératoire.

3. Dispositif selon la revendication 2, dans lequel ledit robot comprend quatre segments articulés qui sont mobiles l'un par rapport à l'autre et permettent auxdits moyens d'irradiation de se déplacer avec six degrés de liberté.

4. Dispositif selon la revendication 1, dans lequel ladite pluralité de cavités de ladite structure d'accélération sont interconnectées au moyen de soudures au laiton imperméables à l'air, les cinq premières cavités possédant une extension croissante, les cavités successives étant d'extension identique, ladite pluralité de cavités produisant une auto-focalisation du faisceau électronique.

5. Dispositif selon la revendication 4, dans lequel ladite structure d'accélération comprend une cathode située en face de ladite première cavité et une lamelle en titane située hors de la dernière cavité.

6. Dispositif selon la revendication 1, dans lequel lesdits moyens de modulation comprennent un magnétron, un modulateur de radiofréquence, et un modulateur de cathode adapté pour habiliter ladite cathode.

7. Dispositif selon la revendication 4, dans lequel ladite structure d'accélération comprend une lamelle en titane située hors de la dernière cavité.

8. Dispositif selon la revendication 1, dans lequel lesdits moyens de modulation comprennent un magnétron et un modulateur de radiofréquence placés dans un bras de la structure articulée.

9. Dispositif selon la revendication 6, dans lequel lesdits moyens de modulations comprennent un contrôle automatique de la fréquence pour maintenir la fréquence d'oscillation du magnétron en coïncidence avec celle de la résonance de la structure d'accélération.
